(19) Europäisches Patentamt
European Patent Office
Office européen des brevets

(11) Publication number: **0 061 882**
**A1**

(12) # EUROPEAN PATENT APPLICATION

(21) Application number: 82301484.0

(22) Date of filing: 23.03.82

(51) Int. Cl.[3]: C 07 D 261/14
C 07 D 271/06

(30) Priority: 27.03.81 US 248380
09.11.81 US 319176

(43) Date of publication of application:
06.10.82 Bulletin 82/40

(84) Designated Contracting States:
BE CH DE FR GB IT LI LU NL SE

(71) Applicant: ELI LILLY AND COMPANY
307, East McCarty Street
Indianapolis Indiana 46285(US)

(72) Inventor: Lechleiter, John Clifford
5547 Carrollton
Indianapolis Indiana 46220(US)

(74) Representative: Crowther, Terence Roger et al,
Erl Wood Manor
Windlesham Surrey GU20 6PH(GB)

(54) **Process for preparing isoxazol-3-ylcarboxamides.**

(57) Isoxazol-3-ylureas of formula I

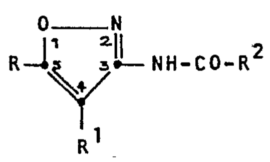

1

wherein R is $C_1$-$C_6$ alkyl, phenyl, or phenyl substituted with 1-2 halo, $C_1$-$C_3$ alkyl or $C_1$-$C_3$ alkoxy groups;

$R^1$ is hydrogen or $C_1$-$C_6$ alkyl;

$R^2$ is $C_1$-$C_6$ alkyl, $-NR^3R^4$, phenyl, or phenyl substituted with 1-2 halo, $C_1$-$C_3$ alkyl or $C_1$-$C_3$ alkoxy groups;

$R^3$ and $R^4$ are independently $C_1$-$C_6$ alkyl; or $R^3$ and $R^4$ combine with the nitrogen atom to which they are bonded to form morpholino, pyrrolidino or piperidino; are prepared in a single synthetic step from 3-methyl-5-substituted-1,2,4-oxadiazoles or 3-acyl-1,2,4-oxadiazoles. Novel 3-acyl-1,2,4-oxadiazole intermediates are also described.

EP 0 061 882 A1

Croydon Printing Company Ltd

PROCESS FOR PREPARING ISOXAZOL-3-YLCARBOXAMIDES

This invention concerns the field of agricultural chemistry, and provides a novel process for preparing useful isoxazoles in a single synthetic step. The compounds prepared by this process were described by Yukinaga in U.S. Patent 4,062,861 and are useful as herbicides. A particularly useful compound of this class is 3,3-dimethyl-1-(5-*t*-butylisoxazol-3-yl)urea, which compound is being tested on a large scale as an herbicide. Intermediates which are formed in this process are 5-substituted-3-acyl-1,2,4-oxadiazoles, which are also herbicides.

The above-mentioned Patent 4,062,861 is the most important document which discloses and explains the compounds of formula I below, but that document shows only the traditional methods for preparing the compounds.

Processes having some pertinence to the process of this invention have been disclosed by Fabian et al. in U.S. Patents 3,957,772 and 3,987,038. The former patent shows a process for making a certain pharmaceutical compound having a 5-methylisoxazol-3-ylcarboxamido group, which is prepared by the high temperature reaction in the presence of a base of a compound having a 3-acetylmethyl-1,2,4-oxadiazol-5-yl group. It is not expected that the present claimed compounds would result from any art, including U.S. 3,957,772. Micetich, *Can. J. Chem.* *48*, 2006-15 (1970), shows an attempted carboxylation of the methyl group of 3-methyl-5-phenyl-1,2,4-oxadiazole. The product was not the desired carboxymethyl compound, but 5-butyl-3-methyl-5-phenyl-1,2,4-4H-oxadiazoline, derived from

the butyllithium base. Micetich further confirms the unexpected results of the present process. The 3,987,038 patent shows an opposite process, wherein a 5-methylisoxazol-3-ylcarboxamide is converted to the corresponding 3-acetylmethyl-1,2,4-oxidiazol-5-yl compound. It is clear that this art process is the reverse of, and teaches away from, the present process.

This invention provides a process for preparing an isoxazole of the formula

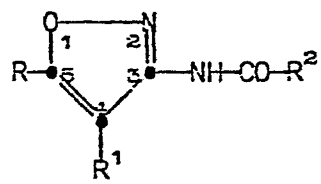

I

wherein R is $C_1$-$C_6$ alkyl, phenyl, or phenyl substituted with 1-2 halo, $C_1$-$C_3$ alkyl or $C_1$-$C_3$ alkoxy groups;

$R^1$ is hydrogen or $C_1$-$C_6$ alkyl;

$R^2$ is $C_1$-$C_6$ alkyl, $-NR^3R^4$, phenyl, or phenyl substituted with 1-2 halo, $C_1$-$C_3$ alkyl or $C_1$-$C_3$ alkoxy groups;

$R^3$ and $R^4$ are independently $C_1$-$C_6$ alkyl; or $R^3$ and $R^4$ combine with the nitrogen atom to which they are bonded to form morpholino, pyrrolidino or piperidino;

which comprises reacting an oxadiazole of the formula

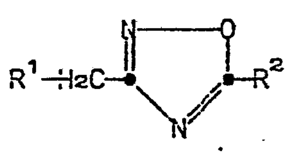

II

wherein $R^1$ and $R^2$ are defined as above;

with an ester of the formula

$$R\text{-}CO_2(C_1\text{-}C_4\text{ alkyl})$$

wherein R is defined as above; in an inert organic solvent in the presence of a very strong base at a temperature from about -100° to about 0° to prepare an acyloxadiazole of the formula

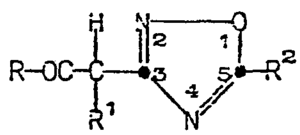

III

wherein R, $R^1$ and $R^2$ are defined as above; and warming the mixture to a temperature from about the ambient temperature to about 100°.

The invention also provides the novel acyloxadiazoles, formula III above, which are intermediates in the process of this invention, and in which the substituents R, $R^1$ and $R^2$ have the meanings described above.

In this document, all temperatures are stated in degrees Celsius, and all expressions of amounts and proportions are in weight units, unless otherwise stated, except for ratios of solvents, which are expressed in volume units.

The general chemical terms used in the above description have their usual meanings in organic chemistry. For example, the terms $C_1$-$C_6$ alkyl, $C_1$-$C_3$ alkyl and $C_1$-$C_3$ alkoxy refer to groups such as methyl, ethyl, propyl, ethoxy, isopropoxy, methoxy, t-butyl,

butyl, pentyl, 3-methylpentyl, 1,1-dimethylpropyl, and hexyl. The word halo refers to chloro, bromo, fluoro and iodo.

A group of typical isoxazoles of formula I prepared by the process of this invention will be mentioned, to assure that the reader of this document fully understands the use of the process of this invention, and the compounds for which the acyloxadiazoles are intermediates.

3-acetamido-5-ethylisoxazole

3-heptanamido-4,5-dimethylisoxazole

4-ethyl-3-(2-methylpropionamido)-5-propylisoxazole

5-isobutyl-4-isopropyl-3-(2-methylbutyramido)isoxazole

5-*s*-butyl-4-pentyl-3-propionamidoisoxazole

4-*t*-butyl-3-(2,2-dimethylbutyramido)-5-pentylisoxazole

4-hexyl-3-hexanamido-5-(2-methylbutyl)isoxazole

3-(2-ethylvaleramido)-5-hexyl-4-(3,3-dimethylbutyl)isoxazole

3-benzamido-4-isobutyl-5-(1,1-dimethylpropyl)isoxazole

3-(2,4-dichlorobenzamido)-5-neopentylisoxazole

3-(3-bromo-5-methylbenzamido)-4-methyl-5-phenylisoxazole

5-(3-chlorophenyl)-4-ethyl-3-(4-ethoxy-2-fluorobenzamido)isoxazole

5-(4-bromophenyl)-5-(2-iodo-4-propylbenzamido)-4-methylisoxazole

5-(3-fluorophenyl)-3-(3,4-dimethylbenzamido)-isoxazole

3-(2-ethyl-4-isopropylbenzamido)-5-(4-iodophenyl)isoxazole

5-(2,5-dichlorophenyl)-3-(3,5-dimethoxybenzamido)isoxazole

5-(2,6-dibromophenyl)-4-propyl-3-(2,4-dipropylbenzamido)isoxazole

5-(2,6-difluorophenyl)-3-(4-methyl-3-propoxybenzamido)isoxazole

3-(4-bromobenzamido)-5-(3,5-diiodophenyl)-isoxazole

4-t-butyl-5-(3-chloro-4-ethylphenyl)-3-(4-chlorobenzamido)isoxazole

5-(4-bromo-2-methylphenyl)-3-(4-fluorobenzamido)isoxazole

5-(3,5-diethylphenyl)-3-(3-iodobenzamido)-4-propylisoxazole

3-(2-ethylbenzamido)-5-(2-methyl-4-propylphenyl)isoxazole

4-isopropyl-5-(4-isopropylphenyl)-3-(4-methylbenzamido)isoxazole

3-(3-isopropylbenzamido)-5-(2,4-dimethylphenyl)isoxazole

3-(4-methoxybenzamido)-5-(2,4-dimethoxyphenyl)isoxazole

4-s-butyl-5-(3-ethoxyphenyl)-3-(4-propoxybenzamido)isoxazole

3-hexyl-3-ethyl-1-[5-(2-methoxy-4-propoxyphenyl)isoxazol-3-yl]urea

3-ethyl-3-methyl-1-[5-(4-ethoxy-3-fluorophenyl)isoxazol-3-yl]urea

1-[5-(2-bromo-4-fluorophenyl)-4-hexylisoxazol-3-yl]-3-ethyl-3-methylurea

1-[5-(4-ethyl-3-ethoxyphenyl)isoxazol-3-yl]-3,3-diisopropylurea

1-(5-isobutylisoxazol-3-yl)-3,3-dibutylurea

1-(5-ethyl-4-isobutylisoxazol-3-yl)-3-hexyl-3-isopropylurea

1-(5-_s_-butylisoxazol-3-yl)-3-_s_-butyl-3-(2-ethylbutyl)urea

3-_s_-butyl-3-methyl-1-(4-methyl-5-phenylisoxazol-3-yl)urea

3-_t_-butyl-1-[5-(2,4-dimethylphenyl)isoxazol-3-yl]-3-propylurea

1-(5-ethyl-4-pentylisoxazol-3-yl)-3-methyl-3-(3-methylpentyl)urea

5-butyl-3-morpholinocarboxamidoisoxazole

3-piperidinocarboxamido-5-propylisoxazole

5-_t_-butyl-3-pyrrolidinocarboxamidoisoxazole

3-methyl-1-(5-pentylisoxazol-3-yl)-3-propylurea

1-(4,5-dihexylisoxazol-3-yl)-3,3-dimethylurea

3,3-dimethyl-1-[5-(2-methylpentyl)isoxazol-3-yl]urea

1-(5-methylisoxazol-3-yl)-3,3-dimethylurea

1-(5-butyl-4-propylisoxazol-3-yl)-3,3-diethylurea

3,3-dipropyl-1-(5-propylisoxazol-3-yl)urea

1-(4-ethyl-5-isopropylisoxazol-3-yl)-3-pentyl-3-propylurea

3-ethyl-1-(5-hexylisoxazol-3-yl)-3-neopentylurea

1-[5-(3,5-dibromophenyl)-4-*s*-butylisoxazol-3-yl]-3-*t*-butyl-3-methylurea

1-(5-ethylisoxazol-3-yl)-3,3-di-*s*-butylurea

3-ethyl-3-methyl-1-(4-isopropyl-5-pentylisoxazol-3-yl)urea

1-(5-*s*-butyl-4-methylisoxazol-3-yl)-3-methyl-3-(1-methylpentyl)urea

The R, $R^1$, $R^2$, $R^3$ and $R^4$ groups of the isoxazoles described above, of course, are derived from the corresponding groups of the starting oxadiazole and ester, and are also the corresponding groups of the novel intermediate acyloxadiazole. To assure that the reader fully understands the intermediate acyloxadiazoles of formula III, which are an important embodiment of the invention, a group of typical compounds will be mentioned by name.

5-methyl-3-propionylmethyl-1,2,4-oxadiazole

5-hexyl-3-(1-acetylethyl)-1,2,4-oxadiazole

5-isopropyl-3-(1-butyrylpropyl)-1,2,4-oxadiazole

5-*s*-butyl-3-[1-(3-methylbutyryl)-2-methylpropyl]-1,2,4-oxadiazole

5-ethyl-3-[1-(2-methylbutyryl)hexyl]-1,2,4-oxadiazole

3-(1-hexanoyl-2,2-dimethylpropyl)-5-(1,1-dimethylpropyl)-1,2,4-oxadiazole

5-pentyl-3-[1-(3-methylvaleryl)heptyl]-1,2,4-oxadiazole

5-(1-ethylbutyl)-3-(1-heptanoyl-2,2-dimethylpentyl)-1,2,4-oxadiazole

3-[1-(2,2-dimethylbutyryl)-3-methylbutyl]-5-phenyl-1,2,4-oxadiazole

5-(2,4-dichlorophenyl)-3-(3,3-dimethylbutyrylmethyl)-1,2,4-oxadiazole

5-(3-bromo-5-methylphenyl)-3-(1-benzoylethyl)-1,2,4-oxadiazole

3-[1-(4-chlorobenzoyl)propyl]-5-(3-ethoxy-5-fluorophenyl)-1,2,4-oxadiazole

3-[1-(3-bromobenzoyl)ethyl]-5-(4-iodo-3-propylphenyl)-1,2,4-oxadiazole

3-(3-fluorobenzoylmethyl)-5-(2,4-dimethylphenyl)-1,2,4-oxadiazole

5-(4-ethyl-2-isopropylphenyl)-3-(3-iodobenzoylmethyl)-1,2,4-oxadiazole

3-(2,4-dichlorobenzoylmethyl)-5-(2,4-dimethoxyphenyl)-1,2,4-oxadiazole

3-[1-(2,5-dibromobenzoyl)butyl]-5-(2,4-dipropylphenyl)-1,2,4-oxadiazole

3-(3,5-difluorobenzoylmethyl)-5-(2-methyl-3-propoxyphenyl)-1,2,4-oxadiazole

5-(2-bromophenyl)-3-(2,5-diiodobenzoylmethyl)-1,2,4-oxadiazole

5-(4-chlorophenyl)-3-[1-(2-chloro-5-ethylbenzoyl)-2,2-dimethylpropyl]-1,2,4-oxadiazole

3-(3-bromo-4-methylbenzoylmethyl)-5-(3-fluorophenyl)-1,2,4-oxadiazole

3-[1-(2,5-diethylbenzoyl)butyl]-5-(2-iodophenyl)-1,2,4-oxadiazole

5-(4-ethylphenyl)-3-(3-methyl-5-propylbenzoylmethyl)-1,2,4-oxadiazole

3-[1-(4-isopropylbenzoyl)-2-methylpropyl]-5-(2-methylphenyl)-1,2,4-oxadiazole

5-(4-isopropylphenyl)-3-(2,5-dimethylbenzoylmethyl)-1,2,4-oxadiazole

5-(3-methoxyphenyl)-3-(2,4-dimethoxybenzoylmethyl)-1,2,4-oxadiazole

3-[1-(4-ethoxybenzoyl)-2-methylbutyl]-5-(3-propoxyphenyl)-1,2,4-oxadiazole

5-ethylhexylamino-3-(2-methoxy-3-propoxybenzoylmethyl)-1,2,4-oxadiazole

5-ethylmethylamino-3-(3-ethoxy-2-fluorobenzoylmethyl)-1,2,4-oxadiazole

3-[1-(3-bromo-5-fluorobenzoyl)heptyl]-5-ethylmethylamino-1,2,4-oxadiazole

3-(4-ethyl-3-ethoxybenzoylmethyl)-5-diisopropylamino-1,2,4-oxadiazole

5-dibutylamino-3-(3-methylbutyrylmethyl)-1,2,4-oxadiazole

5-hexylisopropylamino-3-(3-methyl-1-propionylbutyl)-1,2,4-oxadiazole

5-[N-s-butyl-N-(2-ethylbutyl)amino]-5-(2-methylbutyryl)-1,2,4-oxadiazole

5-s-butylmethylamino-3-(1-benzoylethyl)-1,2,4-oxadiazole

5-t-butylpropylamino-3-(2,5-dimethylbenzoylmethyl)-1,2,4-oxadiazole

5-[N-propyl-N-(3-methylpentyl)amino]-3-(1-propionylhexyl)-1,2,4-oxadiazole

3-hexanoylmethyl-5-methylpropylamino-1,2,4-oxadiazole

5-diethylamino-3-(1-heptanoylheptyl)-1,2,4-oxadiazole

3-[N-(1-methylbutyl)-N-(1-ethylpropyl)amino]-3-(3-methylhexanoylmethyl)-1,2,4-oxadiazole

3-acetylmethyl-5-dihexylamino-1,2,4-oxadiazole

5-[N-(1-methylpentyl)-N-methylamino]-3-(1-valerylbutyl)-1,2,4-oxadiazole

5-[N,N-bis(s-butyl)amino]-3-butyrylmethyl-1,2,4-oxadiazole

5-[N-ethyl-N-(2-methylpentyl)-5-[1-(2-methylpropionyl)propyl]-1,2,4-oxadiazole

5-diethylamino-3-heptanoylmethyl-1,2,4-oxadiazole

3-[1-(2,4-dibromobenzoyl)-2-methylbutyl]-5-hexylpropylamino-1,2,4-oxadiazole

5-[N,N-bis(t-butyl)amino]-3-propionylmethyl-1,2,4-oxadiazole

5-[N-(isopropyl)-N-(4-methylpentyl)amino]-3-(1-hexanoyl-2-methylpropyl)-1,2,4-oxadiazole

5-dipentylamino-3-[1-(2-methylbutyryl)ethyl]-1,2,4-oxadiazole

It will be understood that certain classes of the acyloxadiazoles of formula III are preferred intermediates, and that the process of this invention is preferably used for producing isoxazoles of formula I of certain preferred classes. For example, the following definitions of the groups R, $R^1$, $R^2$, $R^3$ and $R^4$

are those which define preferred classes of compounds. It will be understood that the preferred definitions below may be combined to constitute further, more limited, classes of preferred acyloxadiazoles and isoxazoles.

a) R is $C_1$-$C_6$ alkyl;
b) R is $C_3$-$C_5$ alkyl;
c) R is $C_4$ alkyl;
d) $R^1$ is hydrogen or methyl;
e) $R^1$ is hydrogen;
f) $R^2$ is $-NR^3R^4$;
g) $R^3$ and $R^4$ are independently $C_1$-$C_3$ alkyl;
h) $R^3$ and $R^4$ are both methyl.

All of the starting compounds and reagents used in the process of this invention are known substances and are readily obtained or prepared by the ordinarily skilled organic chemist.

The reactions described in this document proceed according to the expected stoichiometry. One mole of the oxadiazole and 1 mole of the ester react in the presence of 2 moles of the very strong base to prepare 1 mole of the acyloxadiazole, which converts to 1 mole of the isoxazole. No unexpected excess of any of the reagents is necessary. It is advisable, as usual in organic chemistry, to use a small excess, in the range of about 1% to 25% or thereabout, of less expensive or easier to obtain reagents, to assure that the more expensive reagents are fully used. Large excesses, even up to 10X or more, may be used if desired and are not harmful to the process, but there is no reason or necessity to use large excesses.

The concentration of the reagents in the inert organic solvent is not critical to the process. The reactions have been successfully carried out at a concentration in the range of approximately 1 mole per liter of solvent. Higher or lower concentrations, so long as the concentration is not so high that inconvenient precipitation occurs, may be used as is convenient in a given instance.

No unusual safety precautions are necessary in carrying out the process of this invention. Neither the order of addition of the reagents, nor the rate of addition, is critical to the success of the process. It is preferable to add the oxadiazole of formula II and the ester to a solution of the base, or to add the ester to a mixture of the base and the oxadiazole. The rate of addition must be sufficiently slow that the cooling system of the reactor can maintain the desired temperature, but the rate of addition is not otherwise significant.

The reactor must provide adequate mixing efficiency to avoid localized spots of high concentration of one of the reagents in the reaction mixture. Since the mixture is a solution of moderate viscosity, or a light suspension, the ordinary mixers customarily used in chemical reactors are quite adequate for the process.

The process proceeds in a single reactor in two steps, a low-temperature step and a high-temperature step. The product of the low-temperature step is the novel acyloxadiazole of formula III which is an embodiment of this invention, and it is recovered from

the reaction mixture if the mixture is worked up at low temperature. If the isoxazole of formula I is the desired product, the reaction mixture is heated and the isoxazole is produced in the high-temperature step. The yields in both the low- and the high-temperature steps are extremely high, and essentially no by-products are found when the process is carried out at the preferred conditions explained below.

In the first step, the oxadiazole of formula II and the ester are combined in an inert organic solvent in the presence of a very strong base, preferably in one of the two preferred orders of addition discussed above. The substituents R, $R^1$ and $R^2$ of the starting compounds are those of the desired product of the process, and it is unnecessary to discuss those compounds further, except to note that the ester may be any of the $C_1$-$C_4$ alkyl esters of the alkanoic acid having the desired R residue. It has been found that the isobutyl esters are particularly convenient in large-scale synthesis, and they are therefore the preferred esters.

The preferred organic solvents are ethers, of which tetrahydrofuran is most highly preferred. Also alkyl ethers, such as diethyl ether, diisopropyl ether, and isopropyl butyl ether are useful, and bis ethers, of which 1,2-dimethoxyethane is a representative frequently used in chemical synthesis, may also be used. It should be noted that some amount of alkane solvent usually enters the reaction mixture along with the very strong base, since such compounds are usually

handled as solutions in alkanes, and no ill effects have been found from such additions of alkanes to the reaction mixture.

The preferred very strong bases are lithium diisopropylamide and especially sodium diisopropylamide. Lithium diisopropylamide is formed by the reaction of butyllithium with diisopropylamine, and sodium diisopropylamide is made by reacting sodium metal with diisopropylamine in the presence of isoprene. A preferred class of bases includes the sodium and lithium di($C_1$-$C_4$)alkylamides. Other very strong bases such as are frequently used in organic chemical synthesis may also be used, especially the alkyllithiums, represented by ethyllithium, s-butyllithium, isopropyllithium, butyllithium and t-butyllithium.

The reagents and the base are combined at a low temperature from about -100° to about -10°. The preferred temperature range for the low-temperature step of the process is from about -75° to about -20°, more preferably at from about -75° to about -40°. It is important that the first items added to the reactor, preferably the solvent and the base, or the solvent and the oxadiazole, be chilled down to the desired reaction temperature before the other reagents are added, and that the additions are made with adequate agitation and at such a rate that the temperature of the reaction mixture remains at the desired level and that no localized overheated spots occur in the mixture.

The formation of the acyloxadiazole of formula III is usually rapid. Reaction times in the low temperature step of from about 30 minutes to about

12 hours are adequate; in most cases brief times of about 30-60 minutes are satisfactory.

If the acyloxadiazole is to be isolated, it is necessary to neutralize the base in the reaction mixture, and to quench the reaction, as by the addition of aqueous acid to the reaction mixture. Enough acid should be added to achieve neutrality; it is not necessary to use excess acid. When the reaction mixture has been quenched, which step may be carried out at a higher temperature up to about 0°, the acyloxadiazole is then isolated from the mixture by conventional methods, such as by evaporating the solvent, and then extracting the product from the aqueous remainder with a water-immiscible organic solvent.

It should be noted that the acyloxadiazole, as it exists in the low-temperature reaction mixture, is in a deprotonated form. The hydrogen on the carbon atom attached to the 3-position of the oxadiazole ring is removed by the base, and that position bears a negative charge and is probably bonded to an alkali metal ion derived from the base, that is to say, a lithium atom in the case of a reaction in the presence of a lithium base.

If the process is to be carried on to the isoxazole, the reaction mixture is heated after the addition of the reagents is complete, and the process enters the high-temperature stage. The temperature should be raised to from about the ambient temperature--in the range from about 15° to about 35°--to a maximum of about 100°. A preferred range for the

high-temperature step is from about the ambient temperature to about 65°. Most conveniently, the step may be carried out at the reflux temperature of the mixture, particularly when the reaction is carried out in tetrahydrofuran. The process can be carried out under pressure to raise the boiling point, when it is desired to operate at a temperature above the ambient pressure boiling point of the reaction mixture. The high-temperature reaction is rapid, and has been found usually to be complete within one hour. The reaction time at high temperature is not critical, however, and reaction times from a few minutes to several hours are quite satisfactory.

It should be noted that the formation of isoxazoles wherein $R^2$ is aryl or alkyl is slower than is the formation of those wherein $R^2$ is $-NR^3R^4$. Such compounds need longer high temperature reaction periods, or higher temperatures, to obtain economical yields.

When the high-temperature reaction is over, the isoxazole is isolated by neutralizing the strong base, as by the addition of aqueous acid, and removing the product from the neutralized mixture by conventional methods. It has been found to be convenient to remove the solvent under vacuum, and to extract the product from the aqueous mixture with a water-immiscible organic solvent. However, other isolation procedures will readily occur to organic chemists and can be used as desired.

Of course, since the acyloxadiazole compounds of formula III can be isolated in a stable form after the first low-temperature step, this invention also concerns the one step process of reacting the acyloxadiazole of formula III, preferably in the presence of a base, in a polar organic solvent at a temperature from about the ambient temperature to about 100°C. to obtain the isoxazoles of formula I. Suitable polar organic solvents include $C_1$-$C_4$ alkanols, $C_1$-$C_4$ alkanols with water, tetrahydrofuran, ethyl acetate, methylene dichloride, chloroform, dimethylformamide, dimethylsulfoxide, and acetonitrile. Bases which can be used can be selected from a wide variety of well known bases, such as alkali metal hydroxides, for example potassium or sodium hydroxide, alkali metal carbonates, for example sodium or potassium carbonate, trialkylamines, for example trimethylamine, alkalilithiums, for example butyllithium and alkali metal alkoxides, for example potassium t-butoxide. The preferred bases are the alkali metal bases given above. This reaction proceeds best in the presence of base; however, under temperatures from about 90-250°C. some conversion to the isoxazoles of formula I occurs without base present.

The isoxazole of formula I, prepared as a one step reaction from the acyloxadiazole of formula III, is isolated in the same manner as from the high-temperature two step procedure.

The invention is further explained by the following preparative examples. The products of these examples were identified by standard analytical techniques which are identified in the text of the individual examples.

The first example below illustrates the synthesis and isolation of a typical acyloxadiazole.

Example 1

5-dimethylamino-3-(2,2-dimethylpropionylmethyl)-1,2,4-oxadiazole

Lithium diisopropylamide was prepared by the addition of 12.6 ml. of 1.6-molar butyllithium solution in hexane at 0° to 2.9 ml. of diisopropylamine in 20 ml. of tetrahydrofuran. The mixture was stirred at 0° for one hour, and then cooled to -78°. A solution of 1.26 g. of 5-dimethylamino-3-methyl-1,2,4-oxadiazole and 4.0 ml. of methyl pivalate in 10 ml. of tetrahydrofuran was then added over a period of 28 minutes at constant temperature, and rinsed in with an additional 2 ml. of tetrahydrofuran. The mixture was stirred at -78° for 30 minutes, and then the flask was warmed in an ice-bath for 30 minutes. The reaction was then quenched by addition of 42 ml. of cold 1N hydrochloric acid, and the solvent was removed under vacuum. The aqueous mixture was then diluted with 75 ml. of diethyl ether, the layers were separated, and the aqueous phase was extracted again with 25 ml. of diethyl ether. The organic phases were combined, washed with 20 ml. each of water and saturated aqueous sodium chloride, and dried over sodium sulfate. The organics were then concentrated under vacuum to obtain 2.0 g. of a yellow solid, which was identified by nuclear magnetic resonance (nmr) analysis as the desired product in essentially pure form. Recrystallization from diethyl ether gave colorless plates, m.p. 73.5-74°; proton nmr ($CDCl_3$): 3.75 (s, 2H), 3.11 (s, 6H), 1.22 (s, 9H).

The following group of examples illustrate the synthesis of the preferred isoxazole of this invention at various temperatures.

Example 2

1-(5-t-butylisoxazol-3-yl)-3,3-dimethylurea

A 12.6 ml. portion of 1.6-molar butyllithium solution in hexane was reacted with 2.9 ml. of diisopropylamine at 0° in 20 ml. of tetrahydrofuran, and the mixture was stirred at 0° for 105 minutes. To it was added 1.3 g. of 5-dimethylamino-3-methyl-1,2,4-oxadiazole, 4.0 ml. of methyl pivalate and 10 ml. of tetrahydrofuran, over a period of 30 minutes, and the mixture was stirred for 30 minutes more, maintaining the temperature of the mixture at -78°. At this point the acyloxadiazole, 5-dimethylamino-3-(2,2-dimethylpropionylmethyl)-1,2,4-oxadiazole, was formed. The mixture was then warmed to 0° for 75 minutes, and then was warmed to and held at ambient temperature for 1 hour. To the mixture was then added a few ml. of water. Most of the solvent was removed under vacuum, and 30 ml. of 1N hydrochloric acid was added. The mixture was then extracted with 100 ml. of diethyl ether and then with 50 ml. of diethyl ether, and the organic layers were combined and washed with 20 ml. of water and then with 20 ml. of saturated aqueous sodium chloride. Concentration of the organic phase under vacuum and vacuum drying of the residue gave 2.0 g. of an orange crystalline product, which was found by nmr analysis to contain about 80% of the desired product.

Recrystallization from ether gave the pure isoxazole, m.p. 119-121°; proton nmr ($CDCl_3$): 9.02 (bs, 1H), 6.58 (s, 1H), 3.03 (s, 6H), 1.30 (s, 9H).

## Example 3

1-(5-t-butylisoxazol-3-yl)-3,3-dimethylurea

The process was run according to the process of Example 2, except that the oxadiazole and ester were added over a period of 37 minutes, and the mixture was stirred for 30 minutes at -78° to prepare the acyloxadiazole, 5-dimethylamino-3-(2,2-dimethylpropionyl-methyl)-1,2,4-oxadiazole. The mixture was then stirred for 30 minutes each at 0°, at ambient temperature and at 55-60°. The mixture was then cooled to ambient temperature, quenched with 42 ml. of 1N hydrochloric acid, freed of solvent by evaporation, and worked up according to the process of Example 2 to obtain 2.0 g. of a yellow solid which appeared to be essentially pure product according to nmr analysis, substantially identical to the product of Example 2.

Analysis of the product by an analytical high pressure liquid chromatography system (Waters Associates C-18 μ Bondapack, 70% methanol-water; 220 nm detector) indicated that the product was 95.2% pure.

## Example 4

1-(5-t-butylisoxazol-3-yl)-3,3-dimethylurea

The process was run substantially according to the process of Example 2, except that the addition of oxadiazole and ester was over a period of 45-50

minutes at -78°, to form 5-dimethylamino-3-(2,2-dimethylpropionylmethyl)-1,2,4-oxadiazole. As soon as the addition was complete, the flask was placed in an ice-bath and stirred for 30 minutes, then warmed to ambient temperature for 30 minutes, and then heated to 65° for 15 minutes. The mixture was then cooled to 0° and was quenched and worked up as described in Example 2 to obtain 1.9 g. of product, which was confirmed to be the desired product, substantially identical to the product of Example 2, by nmr analysis.

HPLC analysis indicated that the product was 86.2% pure.

Example 5

1-(5-t-butylisoxazol-3-yl)-3,3-dimethylurea

The process was run according to the general process of Example 2, except that the mixture was held at 0° while the oxadiazole and ester were added to it over a period of 40 minutes to form the same acyloxadiazole as prepared in Example 2. The mixture was stirred at 0° for 40 minutes more after the addition was complete, then warmed to ambient temperature for 40 minutes, and then heated to 65° for a few minutes, and finally cooled to 40° over a period of 30 minutes. The mixture was then worked up as described in Example 2 to obtain 1.8 g. of a residue which was found to contain 48.3% of the desired product, as described in Example 2, by HPLC analysis, a result which was confirmed by nmr analysis.

## Example 6

1-(5-t-butylisoxazol-3-yl)-3,3-dimethylurea

The process of Example 2 was followed again, except that the mixture was maintained at from -15° to -12° while the oxadiazole and ester were added over a period of 20 minutes, and the mixture was then allowed to warm to ambient temperature over a period of 90 minutes. The mixture was then heated to 65°, and was then cooled, quenched and worked up as described in Example 2. The product after workup and evaporation of the solvent was 2.1 g. of a residue found by nmr analysis to comprise the desired product, as described in Example 2, and to be 70.5% pure by HPLC analysis.

## Example 7

1-(5-t-butylisoxazol-3-yl)-3,3-dimethylurea

The procedure of Example 4 was varied by reducing the amount of methyl pivalate to 1.6 ml., and adding the oxadiazole-ester solution over a period of 33 minutes. The procedure was otherwise identical to that of Example 4, and the product was found to be 2.2 g. of the desired product, as described in Example 2, and which was found to be 93.9% pure by HPLC analysis.

## Example 8

1-(5-t-butylisoxazol-3-yl)-3,3-dimethylurea

The process was run substantially according to that of Example 7, except that the reaction mixture was held at -12° while the oxadiazole and ester were added over a period of 23 minutes to form 5-dimethylamino-3-(2,2-dimethylpropionylmethyl)-1,2,4-oxadiazole. The mixture was then warmed to 0° over a period of 1 hour, then to 10° over an additional hour, and was then heated quickly to 50° and cooled to ambient temperature over 30 minutes. The mixture was then cooled slightly and worked up as described in Example 7 to obtain 1.9 g. of an orange solid which was found by HPLC analysis to contain 83.5% of the desired product, substantially identical to the product of Example 2.

Example 9

1-(5-t-butylisoxazol-3-yl)-3,3-dimethylurea

The process of Example 8 was repeated, except that the amount of tetrahydrofuran in the preparation of the base was reduced to 10 ml. instead of 20 ml. The addition of oxadiazole and ester to form the desired intermediate acyloxadiazole, 5-dimethylamino-3-(2,2-dimethylpropionylmethyl)-1,2,4-oxadiazole, was over a period of 18 minutes. The mixture was then heated, cooled and worked up as described in Example 8 to obtain 1.9 g. of a light orange powder, which was found by HPLC to be 91.8% pure product, as described in Example 2.

Example 10

1-(5-t-butylisoxazol-3-yl)-3,3-dimethylurea

Sixty-eight ml. of 1.6-molar butyllithium solution in hexane was combined with 15.4 ml. of diisopropylamine and 50 ml. of tetrahydrofuran at -12°, and was allowed to react for 35 minutes. To it was then

added a solution of 6.4 g. of 5-dimethylamino-3-methyl-1,2,4-oxadiazole and 8.1 g. of isobutyl pivalate in 25 ml. of tetrahydrofuran over a period of 30 minutes at -12° to form the acyloxadiazole, 5-dimethylamino-3-(2,2-dimethylpropionylmethyl)-1,2,4-oxadiazole. The mixture was then warmed to ambient temperature and was stirred overnight before being heated briefly to 50°. It was then cooled, was acidified with 40 ml. of 6N hydrochloric acid, and the solvent was removed under vacuum. The aqueous residue was diluted with 25 ml. of water and filtered, and the filter cake was washed with water and dried in vacuum to obtain 8.4 g. of product. The product was confirmed by nmr to comprise the desired product, as described in Example 2, in fairly impure form.

Example 11

1-(5-*t*-butylisoxazol-3-yl)-3,3-dimethylurea

Fourteen ml. of 1.6-molar butyllithium in hexane was combined with 3.2 ml. of diisopropylamine in 10 ml. of tetrahydrofuran at 0°, and the mixture was stirred for 30 minutes at constant temperature. To the mixture was added 1.3 g. of 5-dimethylamino-3-methyl-1,2,4-oxadiazole and 1.8 g. of isobutyl pivalate in 5 ml. of tetrahydrofuran over a period of 30 minutes at 0°, forming the intermediate 5-dimethylamino-3-(2,2-dimethylpropionylmethyl)-1,2,4-oxadiazole. The mixture was then warmed to ambient temperature over 30 minutes, and then was heated quickly to 50°, cooled and worked up according to the process of Example 8 to obtain 2.2 g. of an oil, which was analyzed by nmr

analysis to confirm that it consisted largely of the desired product, substantially identical to the product of Example 2. HPLC analysis indicated that the product was 76.7% pure 1-(5-t-butylisoxazol-3-yl)-3,3-dimethylurea.

Example 12

1-(5-t-butylisoxazol-3-yl)-3,3-dimethylurea

The process of this example was carried out substantially according to that of Example 11, except that the temperature during the addition of the oxadiazole-ester solution was -10°. The mixture was worked up in the same manner as described in Example 11 to obtain 2.3 g. of a semi-solid, which was found by HPLC analysis to contain 91.2% of the desired product as described in Example 2.

Example 13

1-(5-t-butylisoxazol-3-yl)-3,3-dimethylurea

Fourteen ml. of 1.6-molar butyllithium solution in hexane was added to 20 ml. of tetrahydrofuran, and was cooled to -78°. To it was added a mixture of 1.3 g. of 5-dimethylamino-3-methyl-1,2,4-oxadiazole and 4.0 ml. of methyl pivalate in 10 ml. of tetrahydrofuran over 45-50 minutes to form 5-dimethylamino-3-(2,2-dimethylpropionylmethyl)-1,2,4-oxadiazole. The mixture was allowed to warm to -55° over a period of 90 minutes after the addition, and then the reaction flask was warmed to 0° for 45 minutes, before stirring at ambient temperature for 1 hour. To the mixture was then added 25 ml. of 1N hydrochloric acid, and the

solvent was removed under vacuum. The aqueous residue was then extracted with 100 ml. of diethyl ether, and the organic layer was washed with 20 ml. portions of water and saturated aqueous sodium chloride, dried over magnesium sulfate and concentrated under vacuum to obtain 2.6 g. of oil, which was found by nmr analysis to contain only a small amount of the desired product as described in Example 2.

## Example 14

1-(5-*t*-butylisoxazol-3-yl)-3,3-dimethylurea

The process of Example 13 was repeated, except that the base was 16 ml. of 1.3-molar *s*-butyllithium solution in hexane. The product was 2.0 g. of oil, found by nmr analysis to contain a considerable amount of the desired product, as described in Example 2.

## Example 15

1-(5-*t*-butylisoxazol-3-yl)-3,3-dimethylurea

A mixture of 11.5 ml. of 1.8-molar *t*-butyllithium solution in hexane, and 10 ml. of tetrahydrofuran was cooled to -75°, and to it was added a solution of 1.3 g. of 5-dimethylamino-3-methyl-1,2,4-oxadiazole and 2.3 g. of isobutyl pivalate in 20 ml. of tetrahydrofuran at constant temperature over 52 minutes to form 5-dimethylamino-2,2-dimethylpropionylmethyl)-1,2,4-oxadiazole. The mixture was allowed to warm slowly to 10° over a period of 2 hours, 53 minutes. The mixture was then stirred at ambient temperature for 69 hours, and to it was added 25 ml. of 1N hydrochloric acid.

The solvent was removed under vacuum, and the aqueous residue was extracted with 75 ml. and 25 ml. portions of diethyl ether. The combined organics were washed with 25 ml. portions of water and saturated aqueous sodium chloride, and evaporated under vacuum to obtain 2.3 g. of an oil, which was found to consist in large part of the desired product, substantially identical to the product of Example 2.

Example 16

1-(5-*t*-butylisoxazol-3-yl)-3,3-dimethylurea

The process was carried out according to that of Example 15 above, except that the base was 17 ml. of 1.3-molar *s*-butyllithium solution. After the reaction mixture had warmed to 10° as described in Example 15, it was warmed to ambient temperature over 20 minutes, and then heated for 30 minutes at 60-70°. The mixture was then worked up as described in Example 15 to obtain 2.3 g. of orange oil, which was confirmed by nmr analysis to contain a substantial amount of the desired product, as described in Example 2.

Example 17

3-benzamido-5-*t*-butylisoxazole

A 12.6 ml. portion of 1.6-molar butyllithium solution in hexane was added to 2.9 ml. of diisopropylamine and 20 ml. of tetrahydrofuran at 0° and stirred at that temperature for 1 hour. The mixture was then cooled to -78°, and to it was added a solution of 1.6 g. of 3-methyl-5-phenyl-1,2,4-oxadiazole and

3.0 ml. of methyl pivalate in 10 ml. of tetrahydrofuran at constant temperature over a period of 10 minutes. The mixture was then stirred for 30 minutes at constant temperature to form the acyloxadiazole, 3-(2,2-dimethylpropionylmethyl)-5-phenyl-1,2,4-oxadiazole. m.p. 73.5-75.5°; proton nmr ($CDCl_3$): 7.9-8.2 (m, 2H), 7.35-7.6 (m, 3H), 4.0 (s, 2H), 1.25 (s, 9H). The mixture was then warmed to 0° and stirred at that temperature for 90 minutes, and then stirred at ambient temperature for 16 hours. Most of the solvent was removed under vacuum, 40 ml. of 1N hydrochloric acid was added, and the mixture was extracted with 100 ml. and with 25 ml. of diethyl ether. The combined organics were washed with 15 ml. portions of water and of saturated aqueous sodium chloride, dried over sodium sulfate and concentrated under vacuum to obtain 2.6 g. of an oil, which was shown by nmr analysis to contain a substantial amount of the desired product, as described in Example 18.

## Example 18

### 3-benzamido-5-*t*-butylisoxazole

The same starting materials used in Example 17 were used again, except that the amount of the methyl pivalate was only 2.0 ml. The addition of the starting compounds to the base was over a period of 30 minutes to prepare the intermediate 3-(2,2-dimethylpropionylmethyl)-5-phenyl-1,2,4-oxadiazole. The reaction mixture was then warmed to ambient temperature, and was then stirred under reflux for 90 minutes. The mixture was worked up as described in Example 17 to obtain

2.2 g. of a solid, which was purified by chromatography on silica gel, eluting with 5% diethyl ether in dichloromethane. Concentration of the product-containing fractions gave 660 mg. of purified product, m.p. 187-190.5°; proton nmr ($CDCl_3$): 10.22 (bs, 1H), 7.90-8.10 (m, 2H), 7.36-7.61 (m, 3H), 6.89 (s, 1H), 1.37 (s, 9H).

Example 19

5-t-butyl-3-(2,2-dimethylpropionylmethyl)-1,2,4-oxadiazole

5-t-butyl-3-(2,2-dimethylpropionamido)isoxazole

A 12.6 ml. portion of 1.6-molar butyllithium solution in hexane was combined with 2.9 ml. of diisopropylamine and 20 ml. of tetrahydrofuran as described in Example 19, and to it was added 1.4 g. of 5-t-butyl-3-methyl-1,2,4-oxadiazole and 3.0 ml. of methyl pivalate in 10 ml. of tetrahydrofuran. The process was carried out and the product worked up as described in Example 17 to give 2.2 g. of a solid which was shown by nmr analysis to comprise a large amount of the desired oxadiazole. Crystallization from petroleum ether gave the pure compound, m.p. 30.0-30.5°; proton nmr ($CDCl_3$): 3.93 (s, 1H), 1.42 (s, 9H), 1.23 (s, 9H).

The nmr analysis showed that the crude product contained a small amount of the isoxazole, as described in Example 20.

Example 20

5-t-butyl-3-(2,2-dimethylpropionamido)isoxazole

Lithium diisopropylamide was prepared by the reaction of 13.5 ml. of 1.6-molar butyllithium solution

in hexane with 3.0 ml. of diisopropylamine in 25 ml. of tetrahydrofuran at -78°. The mixture was stirred for 15 minutes at constant temperature, and then a solution of 1.4 g. of 5-t-butyl-3-methyl-1,2,4-oxadiazole and 2.0 ml. of methyl pivalate in 10 ml. of tetrahydrofuran was added at constant temperature over 30 minutes. The intermediate 5-t-butyl-3-(2,2-dimethylpropionylmethyl)-1,2,4-oxadiazole formed, m.p. 30.0-30.5° and the reaction mixture was then allowed to warm to ambient temperature and was heated to reflux and stirred at that temperature for 2.5 hours. The mixture was then made acidic with 50 ml. of 1N hydrochloric acid and worked up by ether extraction as described in the examples above to obtain 2.2 g. of a yellow product, which was shown by nmr analysis to consist of about 75% of the desired isoxazole and about 25% of the intermediate acyloxadiazole. Trituration with petroleum ether gave the pure isoxazole as a white powder, m.p. 171-172°; proton nmr ($CDCl_3$): 8.99 (b, s, 1H), 6.74 (s, 1H), 1.33 (s, 9H), 1.31 (s, 9H).

Example 21

3,3-dimethyl-1-(5-phenylisoxazol-3-yl)urea

The strong base was prepared as described above in Example 20, and to it was added a solution of 1.3 g. of 5-dimethylamino-3-methyl-1,2,4-oxadiazole and 1.6 ml. of methyl benzoate in 10 ml. of tetrahydrofuran at -78° over 20 minutes to form the acyloxadiazole, 5-dimethylamino-3-benzoylmethyl-1,2,4-oxadiazole. The mixture was then warmed to ambient temperature, and was then heated to reflux and stirred at that temperature

for 1 hour. The mixture was made acidic with 1N hydrochloric acid, and the resultant precipitate was collected by filtration and vacuum dried. Recrystallization from ethyl acetate-ethanol gave the pure urea as a white powder, m.p. 182-183°; proton nmr (DMSO-$d_6$): 9.52 (bs, 1H), 7.37-7.90 (m, 5H), 7.15 (s, 1H), 2.93 (s, 6H).

Example 22

1-(5-*t*-butylisoxazol-3-yl)-3,3-dimethylurea

To a solution of 16 ml. of diisopropylamine in 50 ml. of tetrahydrofuran at 0°, containing 2.56 g. of freshly cut sodium, was added a solution of 11 ml. of isoprene in 30 ml. of tetrahydrofuran over 75 minutes. The mixture was stirred an additional 135 minutes at 0°, and was then cooled to -70°. To it was added, dropwise, a solution of 6.75 g. of 5-dimethylamino-3-methyl-1,2,4-oxadiazole and 9.55 g. of isobutyl pivalate in 30 ml. of tetrahydrofuran over 40 minutes. The mixture was then allowed to warm slowly to ambient temperature and was stirred for 16 hours. The mixture was then cooled in an ice-bath and to it was added 50 ml. of 5N hydrochloric acid. The solvent was evaporated under vacuum, and the resulting aqueous slurry was filtered. The solids were washed with water and dried under vacuum to obtain 8.95 g. of the desired product, identical to the product of Example 2. HPLC analysis indicated the product was 95.0% pure.

The following group of examples illustrate the synthesis of the preferred isoxazoles of this invention starting from the acyloxadiazoles.

Example 23

1-(5-t-butylisoxazol-3-yl)-3,3-dimethylurea

Potassium hydroxide, 0.97 g., was dissolved in 15 ml. of ethanol, and then 2.0 g. of 5-dimethylamino-3-(2,2-dimethylpropionylmethyl)-1,2,4-oxadiazole was added. The mixture was stirred at ambient temperature for 1.5 hours, and 40 g. of ice water added. The resulting suspension was filtered, and the residue air dried to yield 1.88 g. (85%) of the product. The product is identical to that of Example 2.

Example 24

1-(5-t-butylisoxazol-3-yl)-3,3-dimethylurea

A mixture consisting of 0.49 g. of 5-dimethylamino-3-(2,2-dimethylpropionylmethyl)-1,2,4-oxadiazole, 10 ml. of toluene and 0.7 ml. of triethylamine was stirred under nitrogen, at reflux temperature, for seven hours. The mixture stood overnight at ambient temperature and washed successively with 6 ml. each of 1N hydrochloric acid and water, dried over sodium sulfate and concentrated to yield 0.52 g. of product, as a yellow oil. The product formed was identical to that of Example 2.

When the above reaction was repeated, omitting the triethylamine, and refluxing for 14.5 hours, about 45% of the desired product was formed.

The isoxazoles of formula I prepared by this process are herbicides, and are used as taught in U.S. Patent 4,062,861. The novel acyloxadiazoles of formula III have also been found to be active as herbicides.

A broad spectrum greenhouse test was run to show the activity of the acyloxadiazoles of formula III. Various application rates and a variety of weed and crop species were used as shown in the following tables.

Square plastic pots were filled with a sterilized sandy loam soil and were planted to seeds of the various crops and weeds. Each pot was individually fertilized.

Test compounds of formula III were applied postemergence to some pots and preemergence to others. Postemergence applications of the compounds were sprayed over the emerged plants about 12 days after the seeds were planted. Preemergence applications were sprayed on the soil the day after the seeds were planted.

Each test compound was dissolved in 1:1 acetone:ethanol at the rate of 2 g. per 100 ml. The solution also contained about 2 g. per 100 ml. of an anionic-nonionic surfactant blend. One ml. of the solution was diluted with deionized water to obtain the dilution desired. The appropriate amount of the solution was applied to each pot.

After the compounds were applied, the pots were moved to the greenhouse, watered as necessary, and observed and rated about 10-13 days after application of the compounds. Untreated control plants were used as standards in every test.

## Table 1

### Preemergence

| Compound | Rate of Appln. lbs/acre | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oat | Velvetleaf | Jimsonweed | Morningglory | Zinnia |
|---|---|---|---|---|---|---|---|---|---|---|---|---|---|---|---|---|---|---|---|---|---|
| 5-dimethyl-amino-3-(2,2-dimethylpropionylmethyl)-1,2,4-oxadiazole | 1 | 1 | 2 | 3 | 3 | 4 | 4 | 2 | 5 | 2 | 3 | 5 | 4 | 4 | 5 | 4 | 3 | 5 | 3 | 5 | 4 |
| | 2 | 3 | 2 | 4 | 3 | 5 | 4 | 2 | 5 | 5 | 4 | 5 | 4 | 4 | 5 | 4 | 4 | 5 | 4 | 5 | 5 |
| | 4 | 3 | 5 | 4 | 4 | 5 | 4 | 3 | 5 | 5 | 4 | 5 | 5 | 4 | 5 | 4 | 4 | 5 | 4 | 5 | 5 |

Scale: 1-5 where 1 = normal plants,
5 = dead plants or no emergence

Table 2

Postemergence

| Compound | Rate of Appln. lbs./acre | Tomato | Barnyard Grass | Mustard | Large Crabgrass | Pigweed | Foxtail | Velvetleaf | Morningglory | Wild Oat | Zinnia |
|---|---|---|---|---|---|---|---|---|---|---|---|
| 5-dimethylamino-3-(2,2-dimethylpropionyl-methyl)-1,2,4-oxadiazole | 1 | 3 | 4 | 5 | 5 | 5 | 4 | 5 | 2 | 2 | 5 |
| | 2 | 5 | 4 | 4 | 5 | 5 | 5 | 5 | 5 | 3 | 5 |
| | 4 | 5 | 4 | 5 | 5 | 5 | 5 | 5 | 4 | 4 | 5 |

Scale: 1-5 where 1 = normal plants,
5 = dead plants or no emergence

CLAIMS

1. An acyloxadiazole of the formula

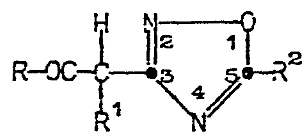

III

wherein R is $C_1-C_6$ alkyl, phenyl, or phenyl substituted with 1-2 halo, $C_1-C_3$ alkyl or $C_1-C_3$ alkoxy groups;

$R^1$ is hydrogen or $C_1-C_6$ alkyl;

$R^2$ is $C_1-C_6$ alkyl, $-NR^3R^4$, phenyl, or phenyl substituted with 1-2 halo, $C_1-C_3$ alkyl or $C_1-C_3$ alkoxy groups;

$R^3$ and $R^4$ are independently $C_1-C_6$ alkyl; or $R^3$ and $R^4$ combine with the nitrogen atom to which they are bonded to form morpholino, pyrrolidino or piperidino.

2. 5-Dimethylamino-3-(2,2-dimethylpropionylmethyl)-1,2,4-oxadiazole.

3. A process for preparing an acyloxadiazole of the formula

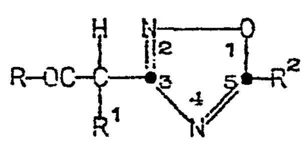

III

wherein R is $C_1-C_6$ alkyl, phenyl, or phenyl substituted with 1-2 halo, $C_1-C_3$ alkyl or $C_1-C_3$ alkoxy groups;

$R^1$ is hydrogen or $C_1-C_6$ alkyl;

$R^2$ is $C_1-C_6$ alkyl, $-NR^3R^4$, phenyl, or phenyl substituted with 1-2 halo, $C_1-C_3$ alkyl or $C_1-C_3$ alkoxy groups;

$R^3$ and $R^4$ are independently $C_1$-$C_6$ alkyl; or $R^3$ and $R^4$ combine with the nitrogen atom to which they are bonded to form morpholino, pyrrolidino or piperidino, which comprises reacting an oxadiazole of the formula

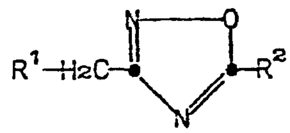

II

wherein $R^1$ and $R^2$ are defined as above; with an ester of the formula

$$R-CO_2(C_1-C_4\ \text{alkyl})$$

wherein R is defined as above; in an inert organic solvent in the presence of a very strong base at a temperature from about -100°C. to about 0°C.

4. A process of claim 3 wherein R is t-butyl, $R^1$ is hydrogen, and $R^2$ is dimethylamino.

5. The process of claim 4 wherein the organic solvent is tetrahydrofuran and the base is sodium diisopropylamide.

6. A process for preparing an isoxazole of the formula

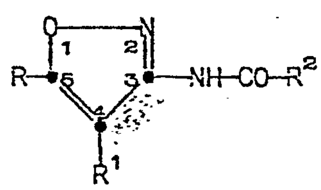

I

wherein R is $C_1$-$C_6$ alkyl, phenyl, or phenyl substituted with 1-2 halo, $C_1$-$C_3$ alkyl or $C_1$-$C_3$ alkoxy groups;

$R^1$ is hydrogen or $C_1$-$C_6$ alkyl;

$R^2$ is $C_1$-$C_6$ alkyl, $-NR^3R^4$, phenyl, or phenyl substituted with 1-2 halo, $C_1$-$C_3$ alkyl or $C_1$-$C_3$ alkoxy groups;

$R^3$ and $R^4$ are independently $C_1$-$C_6$ alkyl; or $R^3$ and $R^4$ combine with the nitrogen atom to which they are bonded to form morpholino, pyrrolidino or piperidino;

which comprises reacting an acyloxadiazole of the formula

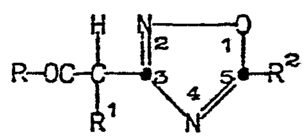

III

wherein R, $R^1$ and $R^2$ are defined as above, preferably in the presence of a base, in a polar organic solvent at a temperature from about the ambient temperature to about 100°C.

7. A process of claim 6 wherein R is t-butyl, $R^1$ is hydrogen and $R^2$ is dimethylamino.

8. The process of claim 7 wherein the base is potassium hydroxide, the polar organic solvent is ethanol, and the temperature is room temperature.

9. A process for preparing an isoxazole of the formula

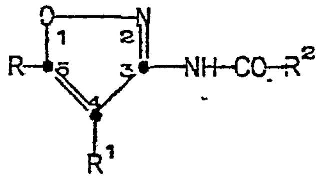

I

wherein R is $C_1$-$C_6$ alkyl, phenyl, or phenyl substituted with 1-2 halo, $C_1$-$C_3$ alkyl or $C_1$-$C_3$ alkoxy groups;

$R^1$ is hydrogen or $C_1$-$C_6$ alkyl;

$R^2$ is $C_1$-$C_6$ alkyl, $-NR^3R^4$, phenyl, or phenyl substituted with 1-2 halo, $C_1$-$C_3$ alkyl or $C_1$-$C_3$ alkoxy groups;

$R^3$ and $R^4$ are independently $C_1$-$C_6$ alkyl; or $R^3$ and $R^4$ combine with the nitrogen atom to which they are bonded to form morpholino, pyrrolidino or piperidino;

which comprises reacting an oxadiazole of the formula

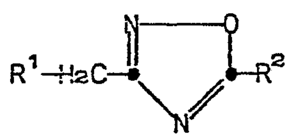

II

wherein $R^1$ and $R^2$ are defined as above;

with an ester of the formula

$$R\text{-}CO_2(C_1\text{-}C_4 \text{ alkyl})$$

wherein R is defined as above;

in an inert organic solvent in the presence of a very strong base at a temperature from about -100° to about 0° to prepare an acyloxadiazole of the formula

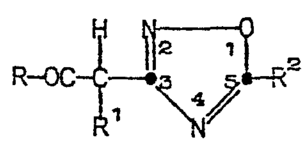

III

wherein R, $R^1$ and $R^2$ are defined as above; and warming the mixture to a temperature from about the ambient temperature to about 100°.

10. A process of claim 9 wherein the ester is a compound wherein R is $C_3$-$C_5$ alkyl.

11. A process of claim 9 wherein the oxadiazole is a compound wherein $R^1$ is hydrogen.

12. A process of claim 9 wherein the oxadiazole is a compound wherein $R^2$ is $-NR^3R^4$ and $R^3$ and $R^4$ are independently $C_1$-$C_3$ alkyl.

13. A process of claim 9 for preparing 1-(5-t-butylisoxazol-3-yl)-3,3-dimethylurea wherein the ester is a compound wherein R is t-butyl and the oxadiazole is 5-dimethylamino-3-methyl-1,2,4-oxadiazole.

14. A process of claim 9 wherein the very strong base is a lithium or sodium di($C_1$-$C_4$ alkyl)-amide.

15. A process of claim 14 wherein the very strong base is lithium diisopropylamide.

16. A process of claim 14 wherein the very strong base is sodium diisopropylamide.

17. A process of claim 9 wherein the inert organic solvent is an ether.

18. A process of claim 17 wherein the ether is tetrahydrofuran.

19. A process of any one of claims 9-18 wherein the oxadiazole is contacted with the ester at a temperature from about -75° to about -20°.

20. A process of any one of claims 9-18 wherein the mixture is warmed to a temperature from about the ambient temperature to about 65°.

0061882

European Patent Office

# EUROPEAN SEARCH REPORT

Application number

EP 82 30 1484

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | JOURNAL OF HETEROCYCLIC CHEMISTRY, vol.15, December 1978, Hetero Corp., Provo, Utah (US) H.L. Yale et al.:"3,5-Disubstituted-1,2,4-oxadiazoles and 4,5-dihydro-3,5-disubstituted-1,2,4-oxadiazoles", pages 1373-1378 * page 1373, compound 4, page 1374, compound 2, pages 1376-1377 * --- | 1 | C 07 D 261/14<br>C 07 D 271/06 |
| D,A | US-A-3 957 772 (ARTHUR C. FABIAN) * claim 1 * ----- | 6 | |
| | | | TECHNICAL FIELDS SEARCHED (Int. Cl. 3) |
| | | | C 07 D 261/00<br>C 07 D 271/00 |

The present search report has been drawn up for all claims

| Place of search | Date of completion of the search | Examiner |
|---|---|---|
| THE HAGUE | 16-06-1982 | HENRY J.C. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document